# EUROPÄISCHE PATENTSCHRIFT

(11) **EP 0 693 559 B1**
(45) Veröffentlichungstag und Bekanntmachung des Hinweises auf die Patenterteilung: **12.09.2001**
(21) Anmeldenummer: 95111047.7
(22) Anmeldetag: 14.07.1995
(51) Int. Cl.: C12Q 1/37, G01N 33/68, G01N 33/72

(54) **Verfahren zur quantitativen Bestimmung von glykierten Proteinen**
Method for quantitative determination of glycated proteins
Procédé pour la détermination quantitative des protéines glycés

(30) Priorität: 18.07.1994 DE 4425162
(43) Veröffentlichungstag der Anmeldung: 24.01.1996
(73) Patentinhaber: Roche Diagnostics GmbH, 68298 Mannheim (DE)
(72) Erfinder: Kobold, Uwe, Dr., D-82362 Weilheim (DE); Renauer, Doris, Dr., D-82166 Graefeling (DE); Finke, Andreas, Dr., D-82377 Penzberg (DE); Karl, Johann, Dr., D-82380 Peissenberg (DE); Jeppsson, Jan-Olof, Prof. Dr., S-21620 Malmö (SE)

(56) Entgegenhaltungen:
- EP-A- 0 526 150
- HEMOGLOBIN, Bd.17, Nr.4, April 1993, NEW YORK, NY, US Seiten 303 - 318 B. LANDLIN AND J-O. JEPPSSON 'Rare .beta.-Chain Hemoglobin Variants found in Swedish Patients During HbA1c Analysis'
- CARBOHYDR. RES. (1985), 138(2), 305-14 CODEN: CRBRAT;ISSN: 0008-6215, 1985 SCHLUETER, MICHAEL ET AL 'Isolation of glycopeptides containing individual glycosylation by methylation analysis'
- 'Arbeitsanleitung Klinische Chemie' 1992 , E. MERCK AG , DARMSTADT DE *Glycosyliertes Hämoglobin (Glyc-Hb)* * Seite 89 *

## Beschreibung

Die Erfindung betrifft ein Verfahren zur quantitativen Bestimmung von glykiertem Protein, insbesondere Hämoglobin (HbA1c), welches im wesentlichen aus einem enzymatischen und einem chromatographischen Verfahrensschritt besteht. Anschließend wird durch Differenzmessung von beispielsweise glykiertem und nicht glykiertem Hämoglobin (HbA0) der exakte Anteil an HbA1c bestimmt. Alternativ kann der Absolutgehalt von glykierten und nicht-glykierten Proteinen durch Kalibration mit geeigneten Standardmaterialien bestimmt werden.

Zur Bestimmung von glykierten Proteinen gibt es eine Vielzahl von Verfahren. Diese lassen sich prinzipiell in drei Gruppen einteilen, in Abhängigkeit von der Art und Weise, wie glykierte und nichtglykierte Proteinkomponenten getrennt und quantifiziert werden (Clin. Chemistry 32 (1986), B64 - B70). Als erste Gruppe sind physikalisch-chemische Methoden, basierend auf der Nutzung von Ladungsunterschieden, zu nennen. Darunter befinden sich die in der klinischen Chemie am häufigsten verwendeten Verfahren der HPLC-Bestimmung mit KationenaustauscherSäulen, z. B. Diamat, MonoS und PolyCat A (Bisse-Methode). Die quantitative Auswertung erfolgt, beispielsweise im Falle von Hämoglobin, durch die Relativmessung des HbA1c-Signales, bezogen auf die Gesamtmenge Hb (% HbA1c).

Als zweite Gruppe sind Methoden anzuführen, welche die unterschiedliche chemische Reaktivität von glykiertem und nichtglykiertem Protein ausnutzen. Hierzu gehören die Thiobarbitursäure-Methode, bei der beispielsweise die an Hämoglobin gebundene Glucose in einen gelben Farbstoff überführt und photometrisch gemessen wird, aber auch affinitätschromatographische Verfahren, bei denen die Komplexbildung zwischen den vicinalen Diolgruppen des Zuckerrestes und einer Boronsäuregruppierung, welche kovalent an einen Träger gebunden ist, zur Trennung des glykierten und nichtglykierten Hämoglobins verwendet werden. Die Quantifizierung der getrennten Substanzklassen erfolgt photometrisch unter Berechnung des Relativgehaltes von glykiertem Hämoglobin oder im Fall der Thiobarbitursäure-Methode als Absolutbestimmung durch Kalibration mit geeigneten Standardmaterialien.

Drittens sind immunologische Verfahren zu nennen. Bei immunologischen Verfahren werden spezifische Antikörper verwendet. Diese erkennen beispielsweise die für HbA1c typische Struktureinheit des am N-terminalen Ende der β-Kette glykierten Hämoglobin-Moleküls (z. B. Tina quant® HbA1c, Boehringer Mannheim). Bei den immunologischen Verfahren erfolgt eine Bestimmung des Absolutgehaltes des jeweiligen Proteins. Dazu ist die Verwendung von Kalibratoren notwendig, denen durch ein unabhängiges Verfahren eine Sollkonzentration zugewiesen wird. Der relative Gehalt beispielsweise an HbA1c, kann nicht durch eine direkte Messung erhalten werden.

Die bekannten Verfahren sind jedoch mit einer Reihe von Nachteilen verbunden. So zeigen die physikalisch-chemischen Verfahren eine z. T. sehr schlechte Selektivität, da die gemessenen Signale von glykiertem Protein durch die der nichtglykierten Varianten überlagert sind. Die chromatographischen Peakformen sind häufig asymmetrisch und schlecht integrierbar. Die verwendeten Kationenaustauschersäulen sind anfällig gegenüber kleinen Änderungen der Arbeitsbedin gungen und Kontaminationen. Bedingt durch die schlechte Selektivität besteht ein hohes Risiko zu hohe Werte zu messen (falsch-positive Werte).

Bei den chemischen Verfahren ist die Durchführung nur schwierig standardisierbar und Störun gen durch andere zuckerhaltige Komponenten nur mit großem Aufwand zu vermeiden. Eine U₁ terscheidung zwischen beispielsweise HbA1c und anderen glykierten Hämoglobinvarianten ist nicht möglich.

Die immunologischen bzw. affinitätschromatographischen Verfahren zur Bestimmung von glykiertem Protein, wie z.B. Hämoglobin, neben nichtglykiertem Protein zeichnen sich durch eine sehr hohe Selektivität gegenüber glykierten Proteinvarianten aus. Die Qualität der Ergebnisse ist jedoch abhängig von der Qualität der verwendeten Standards zur Kalibration. Geeignet Primärstandards in optimaler Qualität sind zur Zeit, insbesondere für HbA1c, nicht verfügbar. Informationen zu Matrixabhängigkeiten können mangels einer geeigneten Referenzmethode nicht gewonnen werden. Auch hier werden häufig falsch-positive Werte erhalten (siehe z. B. J. Clin. Lab. Anal. 8, (1994), 128 - 134; Arbeitsanleitung Klinische Chemie 1992, E. Merck AG, Darmstadt/Deutschland).

Der Erfindung liegt daher die Aufgabe zugrunde, ein Verfahren zur Verfügung zu stellen, wodurch die Nachteile der bekannten Verfahren überwunden werden. Insbesondere besteht die Aufgabe darin, ein Verfahren bereitzustellen, das nicht von der Qualität von Primärstandards zur Kalibration abhängig ist und falsch-positive Werte unterdrückt.

Die Lösung der Aufgabe besteht darin, daß eine ein glykiertes Protein bzw. entsprechende Varianten enthaltende wäßrige Probe zunächst mit einem proteolytischem Enzym behandelt wird und anschließend die resultierende Mischung mit den Bruchstücken chromatographisch aufgetrennt wird.

Im einzelnen wird dabei wie folgt verfahren:

Das Probenmaterial, insbesondere hämolysiertes Vollblut, wird mit einem proteolytischem Enzym in einem geeigneten Reaktionspuffer inkubiert. Die Hämolyse erfolgt nach bekannten Methoden. Als Enzym haben sich hier solche Proteasen als geeignet erwiesen, die befähigt sind, von der β-Kette des Hämoglobins ein N-terminales, möglichst kurzes Peptid abzuspalten. Neben Trypsin haben sich hier insbesondere Chymotrypsin und Thermolysin und ganz besonders Endoproteinase Glu-C als geeignet erwiesen. Die proteolytischen Enzyme werden üblicherweise in einer Konzentration von 1/200 bis 1/10 der Gewichtsmenge Protein eingesetzt.

Geeignete Reaktionspuffer sind beispielsweise Ammoniumcarbonat- und/oder Natriumphosphat-Puffer, die üblicherweise in Konzentrationen von ungefähr bis 10 mM im pH-Bereich von 3.5 - 8.5 eingesetzt werden und ggf., abhängig von der jeweiligen Protease, durch weitere Hilfsstoffe, wie Aktivatoren, und/oder Stabilisatoren ergänzt werden können, wie z.B. Natriumdodecylsulfat, Harnstoff, Guanidinhydrochlorid oder Acetonitril.

Die Inkubation mit dem jeweiligen proteolytischen Enzym erfolgt zwischen 20°C und 40°C, vorteilhafterweise bei ca. 25°C und kann, je nach Enzym, nach einigen Minuten bis maximal 18 Stunden beendet werden.

Als besonders vorteilhaft hat sich zudem erwiesen, wenn vor der Hämolyse die Erythrozyten abgetrennt wurden und die Hämolyse lediglich mit Wasser durchgeführt wird.

Die durch Enzymbehandlung erhaltene Lösung enthält neben anderen Fragmenten die für die jeweils zu bestimmenden Proteine spezifischen Peptide. Die Peptidgemische werden anschließend in einem HPLC-Verfahren mit guter Auflösung (z.B. Reversed-phase-Materialien) getrennt und photometrisch detektiert. Solche Trennverfahren sind dem Fachmann allgemein bekannt (z.B. K.L. Stone, J.L Elliot, G. Peterson, W. McMurray, K.R. Williams, "Reversed-phase high performance liquid chromatography for fractionation of enzymatic digests and chemical cleavage products of proteins", Methods in Enzymology, 193 (1990) 389-412). Alternativ kann auch mit anderen Trennverfahren, wie z.B. Kapillarelektrophorese gearbeitet werden (z. B. Capillary Electrophoresis Separations of Peptides: Practical Aspects and Applications, Chap. 9, S.237-271 in Capillary Electrophoresis, Academic Press 1992).

Aus den gemessenen Signalintensitäten des glykierten und des jeweiligen nicht-glykierten Peptidanteils ist die gleichzeitige selektive Bestimmung von glykiertem und nichtglykiertem Protein möglich. Bei der rechnerischen Auswertung wird der prozentuale Anteil von glykiertem Protein bezogen auf nichtglykiertes Protein erhalten. Insbesondere ist das Verfahren für die Bestimmung von HbA1c neben nichtglykiertem Hämoglobin (HbA0) von Vorteil. Hier hat sich das Verfahren als hochspezifisch und frei von jeglicher Art von Störungen erwiesen, zudem ist die Bestimmung des prozentualen Gehaltes an HbA1c ohne zusätzliche Kalibration oder Standardisierung möglich.

Die folgenden Beispiele erläutern die Erfindung weiter.

### Beispiel 1:

Die Methode wurde an einem Vollbluthämolysat und an unterschiedlich aufgereinigten HbA1c und HbA0 Standardmaterialien durchgeführt. Zur Quantifizierung von HbA1c und HbA0 wurde jeweils das N-terminale Peptid mit einer Länge von zweiundzwanzig Aminosäuren verwendet.

Zu Vergleichszwecken wurden die verwendeten Proben auch mit einem HPLC-Verfahren auf Kationenaustauscher Basis (PolyCat A, Bisse-Methode) ohne vorherige Enzymbehandlung vermessen.

### A Erfindungsgemäß (Enzym + HPLC)

### A.1 Enzymlösung:

- Endoproteinase Glu-C
   (sequencing grade Boehringer Mannheim; Id.-Nr. 1047817)
   Proteinkonzentration: ca. 1mg/ml
   Verhältnis Protein: Enzym 20:1
- 25 mM Ammoniumacetat-Puffer mit 5% Acetonitril
   pH 4,0
   Enzymbehandlung, Bedingungen: 18h, 25°C

### A.2 HPLC-Schritt, Bedingungen:

HPLC-Anlage HP 1090
Trennsäule Zorbax C8 stable bond
Gradientenelution von Wasser nach Acetonitril mit 0.1 % Trifluoressigsäure (TFA)
Detektion bei 214 mm

### B HPLC-Schritt ohne vorherige Enzymbehandlungen

Säulenmaterial: PolyCat A
Durchführung entsprechend E. Bisse & H. Wieland, J. of Chromat. 434 (1988) 95-110.

Die Ergebnisse zeigen, daß die Selektivität der Kationenaustauscherchromatographie allein nicht den Anforderungen entspricht, es wird eine falsch-positive Abweichung von ca. 15 % gefunden. Bei Probenmaterial, welches durch Affinitätschromatographie aufgereinigt wurde und kein nichtglykiertes Hämoglobin mehr enthält sind die Ergebnisse der beiden Verfahren vergleichbar, da jetzt das HbA1c-Signal in der Kationenaustauscherchromatographie nicht mehr durch nichtglykiertes Material überlagert werden kann.

**Tabelle 1**

| Probe | % HbA1c Glu-C + HPLC | % HbAlc PolyCat A | % Abweichung |
|---|---|---|---|
| Vollblut Hämolysat | 5.5 | 6.5 | 15.4 |
| HbA1c präparativ gereinigt Kationenaustauscher | 7.5 | 89 | 15.7 |
| HbA1c präparativ gereinigt Affinitätschromatographie | 93 | 92 | -1.1 |
| HbA0 präparativ gereinigt Kationenaustauscher | 0 | 0.6 | |

Tabelle 1 zeigt einen Vergleich der erfindungsgemäßen Methode mit einer reinen HPLC-Methode.

### Beispiel 2:

Zur Überprüfung der Reproduzierbarkeit des Verfahrens wurde eine Vollblutprobe mehrfach aufgearbeitet und nach der Enzymbehandlung das N-terminale Peptid mit einer

Länge von 6 Aminosäuren zur Quantifizierung von HbA1c und HbAo verwendet (vgl. Chromatogramm Abb. 5)

### Enyzmlösung:

- Endoproteinase Glu-C
   (sequencing grade Boehringer Mannheim; Id.-Nr. 1047817)
   Proteinkonzentration : ca. 1 mg/ml
   Verhältnis Protein : Enzym 30 : 1 bis 60 : 1
- 25 mM Ammoniumacetat-Puffer pH 4.0

Die Ergebnisse (Tabelle 2) zeigen, daß das Verfahren gut reproduzierbar durchzuführen ist.

**Tabelle 2**

| Probe | Verhältnis Protein: Enzym | % HbA1c (Glu-C + HPLC) |
|---|---|---|
| Vollblut Hämolysat Ansatz 1 | 43 : 1 | 5.35 |
| Vollblut Hämolysat Ansatz 2 | 43 : 1 | 5.38 |
| Vollblut Hämolysat Ansatz 3 | 43 : 1 | 5.48 |
| Vollblut Hämolysat Ansatz 4 | 43 : 1 | 5.25 |
| Vollblut Hämolysat Ansatz 5 | 43 : 1 | 5.44 |
| Vollblut Hämolysat Ansatz 6 | 34 : 1 | 5.39 |
| Vollblut Hämolysat Ansatz 7 | 51 : 1 | 5.50 |

Tabelle 2 zeigt die Ergebnisse zu einem Reproduzierbarkeitsversuch mit der erfindungsgemäßen Lösung. Die Bedingungen sind wie folgt:
HPCL Anlage HP 1090
Trennsäule Zorbax SB-CN
Gradientenelution von Wasser nach Acetonitril mit 0.1 % Trifluoressigsäure Detektion bei 214 nm

### Abbildung 1:

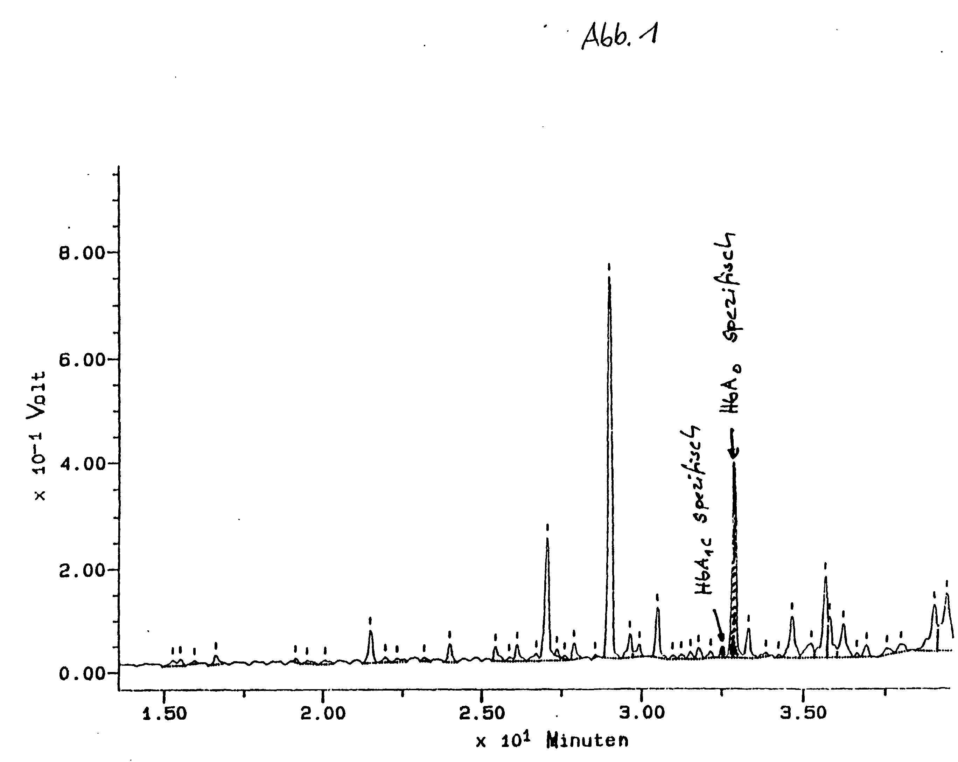

Abbildung 1 zeigt das Ergebnis des erfindungsgemäßen Verfahrens (Probe: Vollblut-Hämolysat; Enzymbehandlung und Chromatographie (HPLC)).

### Abbildungen 2 und 3:

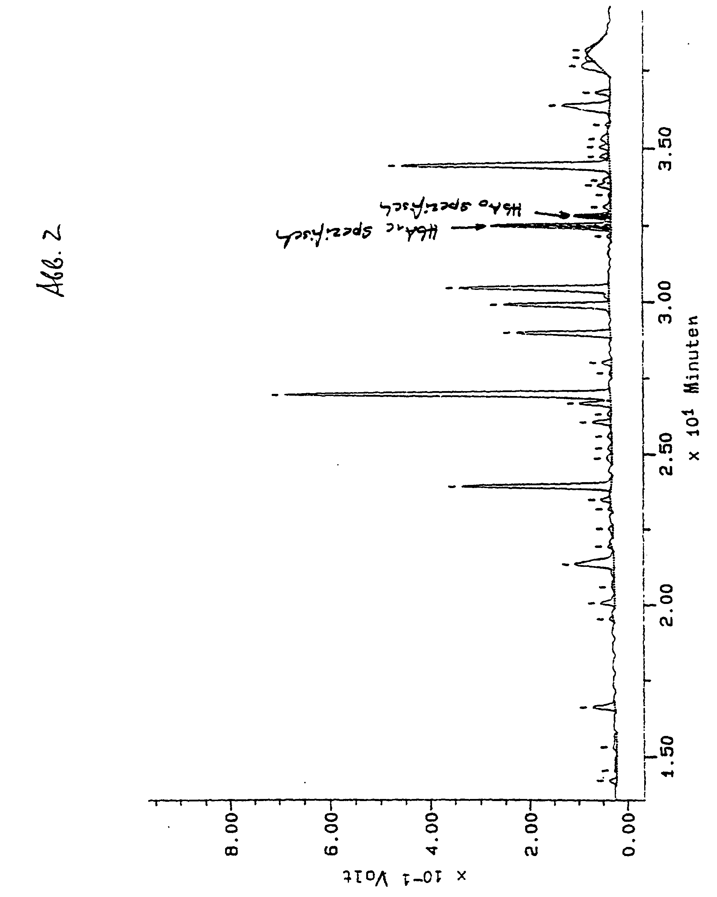

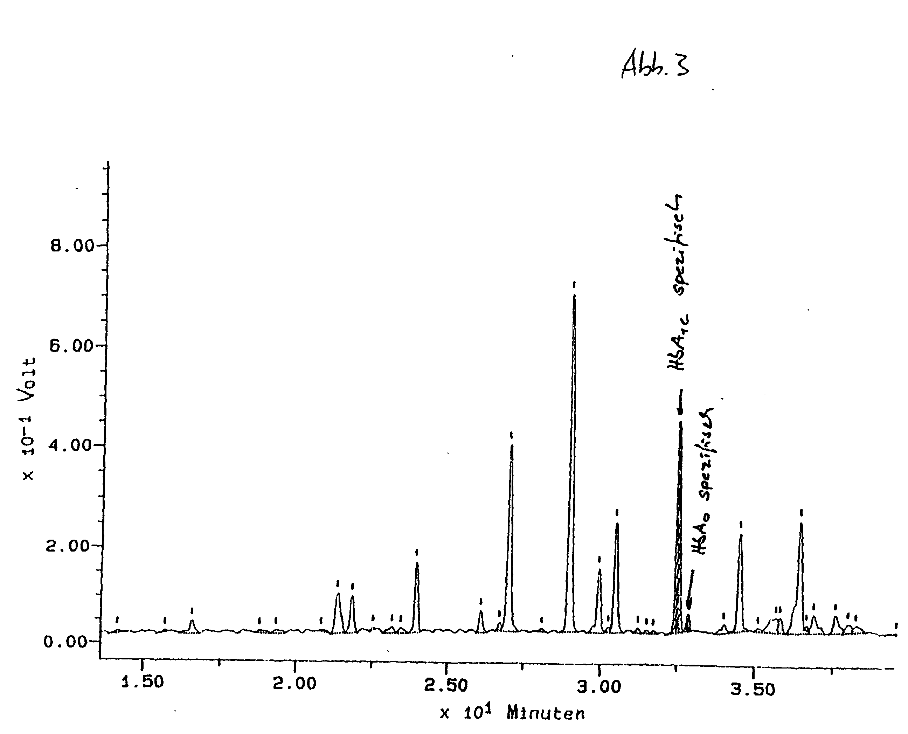

Abbildungen 2 und 3 zeigen das Ergebnis des erfindungsgemäßen Verfahrens (Probe: präparativ gereinigtes HbA1c; 2: Kationenaustauscher; 3: Kationenaustauscher und Affinitätschromatographie).

### Abbildung 4:

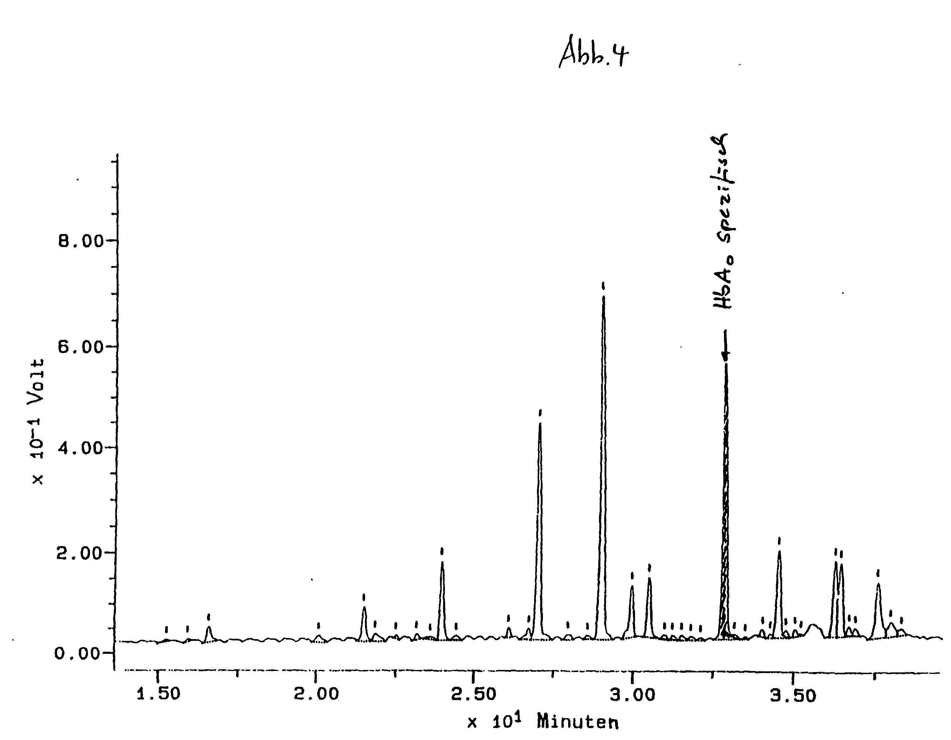

Abbildung 4 zeigt das Ergebnis des erfindungsgemäßen Verfahrens (Probe: präparativ gereinigtes HbA0 (Kationenaustauscher).

Abbildung 4 zeigt das Ergebnis des erfindungsgemäßen Verfahrens (Probe: präparativ gereinigtes HbA0 (Kationenaustauscher).

## Patentansprüche

1. Verfahren zur Bestimmung von glykiertem Protein bzw. entsprechenden Varianten in einer wäßrigen Probe, **dadurch gekennzeichnet, daß** die Probe mit einem proteolytischen Enzym in Kontakt gebracht wird, die entstandene Peptidmischung durch ein nicht immunologisches Verfahren aufgetrennt wird und der Gehalt an glykiertem Protein relativ zu nicht-glykiertem Protein durch Messung der Menge an glykiertem Protein und der Menge an nicht-glykiertem Protein und entsprechendem Vergleich bestimmt wird.

2. Verfahren nach Anspruch 1, **dadurch gekennzeichnet, daß** der Gehalt an glykiertem Protein rechnerisch durch Quotientenbildung aus dem Gehalt an glykiertem Protein und dem Gehalt an nicht-glykiertem Protein bestimmt wird.

3. Verfahren nach Anspruch 1, **dadurch gekennzeichnet, daß** es sich um ein chromatographisches Verfahren handelt.

4. Verfahren nach Anspruch 1 oder 3, **dadurch gekennzeichnet, daß** eine HPLC-Chromatographie oder kapillarelektrophoretische Trennung durchgeführt wird.

5. Verfahren nach einem der vorangegangenen Ansprüche, **dadurch gekennzeichnet, daß** es sich bei der Probe um Vollblut-Hämolysat handelt.

6. Verfahren nach Anspruch 5, **dadurch gekennzeichnet, daß** Erythrozyten vor Inkontaktbringen mit dem proteolytischen Enzym abgetrennt wurden.

7. Verfahren nach einem der vorangegangenen Ansprüche, **dadurch gekennzeichnet, daß** es sich bei dem proteolytischen Enzym um Trypsin, Chymotrypsin, Thermolysin und/oder Endoproteinase Glu-C handelt.

8. Verfahren nach einem der vorangegangenen Ansprüche, **dadurch gekennzeichnet, daß** es sich bei dem glykierten Protein um HbA1c handelt.

9. Verfahren nach Anspruch 1, **dadurch gekennzeichnet, daß** die Probe mit einem proteolytischen Enzym in einem Reaktionspuffer ausgewählt aus der Gruppe bestehend aus Ammoniumcarbonat-, Natriumphosphat- und Ammoniumcarbonat/Natriumphosphatpuffer in Kontakt gebracht wird.

10. Verfahren nach Anspruch 9, **dadurch gekennzeichnet, daß** der Reaktionspuffer einen pH-Wert zwischen 3.5 bis 8.5 aufweist.

11. Verfahren nach Anspruch 9, **dadurch gekennzeichnet, daß** der Reaktionspuffer mindestens einen Aktivator, einen Stabilisator oder einen Aktivator und Stabilisator enthält.

12. Verfahren nach einem der vorangegangenen Ansprüche, **dadurch gekennzeichnet, daß** die Probe mit einem proteolytischen Enzym bei einer Temperatur von 20° bis 40°C in Kontakt gebracht wird.

## Claims

1. Method for the determination of glycated protein and variants thereof in an aqueous sample, wherein the sample is contacted with a proteolytic enzyme, the peptide mixture that is formed is separated by a non-immunological method and the content of glycated protein relative to non-glycated protein is determined by measuring the amount of glycated protein and the amount of non-glycated protein and comparing them.

2. Method as claimed in claim 1, wherein the content of glycated protein is determined mathematically by calculating a quotient from the content of glycated protein and the content of non-glycated protein.

3. Method as claimed in claim 1, wherein it is a chromatographic method.

4. Method as claimed in claim 1 or 3, wherein HPLC chromatography or capillary-electrophoretic separation is carried out.

5. Method as claimed in one of the previous claims, wherein the sample is a whole blood haemolysate.

6. Method as claimed in claim 5, wherein erythrocytes are separated before contact with the proteolytic enzyme.

7. Method as claimed in one of the previous claims, wherein the proteolytic enzyme is trypsin, chymotrypsin, thermolysin and/or endoproteinase Glu-C.

8. Method as claimed in one of the previous claims, wherein the glycated protein is HbA1c.

9. Method as claimed in claim 1, wherein the sample is contacted with a proteolytic enzyme in a reaction buffer selected from the group comprising ammonium carbonate, sodium phosphate and ammonium carbonate/ sodium phosphate buffer.

10. Method as claimed in claim 9, wherein the reaction buffer has a pH value between 3.5 and 8.5

11. Method as claimed in claim 9, wherein the reaction buffer contains at least an activator, a stabilizer or an activator and stabilizer.

12. Method as claimed in one of the previous claims, wherein the sample is contacted with a proteolytic enzyme at a temperature of 20° to 40°C.

## Revendications

1. Procédé pour la détermination de la teneur en protéine glyquée respectivement en variants correspondants d'un échantillon aqueux, **caractérisé en ce qu'**on amène l'échantillon en contact avec une enzyme protéolytique, on sépare le mélange peptidique obtenu via un procédé non immunologique et on détermine la teneur en protéine glyquée par rapport à celle de la protéine non glyquée en mesurant la quantité de protéine glyquée et la quantité de protéine non glyquée et en procédant à une comparaison correspondante.

2. Procédé selon la revendication 1, **caractérisé en ce qu'**on détermine la teneur en protéine glyquée par calcul en formant le quotient de la teneur en protéine glyquée et de la teneur en protéine non glyquée.

3. Procédé selon la revendication 1, **caractérisé en ce qu'**il s'agit d'un procédé chromatographique.

4. Procédé selon la revendication 1 ou 3, **caractérisé en ce qu'**on effectue une chromatographie HPLC ou une séparation par électrophorèse capillaire.

5. Procédé selon l'une quelconque des revendications précédentes, **caractérisé en ce que**, en ce qui concerne l'échantillon, il s'agit de sang entier hémolysé.

6. Procédé selon la revendication 1, **caractérisé en ce qu'**on sépare les érythrocytes avant la mise en contact avec l'enzyme protéolytique.

7. Procédé selon l'une quelconque des revendications précédentes, **caractérisé en ce que**, en ce qui concerne l'enzyme protéolytique, il s'agit de la trypsine, de la chimotrypsine, de la thermolysine et/ou de l'endoprotéinase Glu-C.

8. Procédé selon l'une quelconque des revendications précédentes, **caractérisé en ce que**, en ce qui concerne la protéine glyquée, il s'agit de HbA1c.

9. Procédé selon la revendication 1, **caractérisé en ce qu'**on amène l'échantillon en contact avec une enzyme protéolytique dans un tampon réactionnel choisi parmi le groupe constitué par un tampon de carbonate d'ammonium, de phosphate de sodium et de carbonate d'ammonium/phosphate de sodium.

10. Procédé selon la revendication 9, **caractérisé en ce que** le tampon réactionnel présente une valeur de pH entre 3,5 et 8,5.

11. Procédé selon la revendication 9, **caractérisé en ce que** le tampon réactionnel contient au moins un activateur, un stabilisateur ou bien un activateur et un stabilisateur.

12. Procédé selon l'une quelconque des revendications précédentes, **caractérisé en ce que** l'on amène l'échantillon en contact avec une enzyme protéolytique à une température de 20°C à 40°C.
